# EUROPEAN PATENT SPECIFICATION

(11) **EP 1 244 448 B1**
(45) Date of publication and mention of the grant of the patent: **27.05.2009**
(21) Application number: 00992255.0
(22) Date of filing: 29.11.2000
(51) Int. Cl.: A61K 31/451, A61K 31/4525, A61K 31/454, A61K 31/4748

(54) **NOVEL METHODS FOR THE TREATMENT AND PREVENTION OF ILEUS**
NEUE METHODEN UND VERFAHREN ZUR BEHANDLUNG UND VERMEIDUNG VON ILEUS
NOUVELLES METHODES DE TRAITEMENT ET DE PREVENTION DE L'OCCLUSION INTESTINALE

(30) Priority: 29.11.1999 US 450920
(43) Date of publication of application: 02.10.2002
(73) Proprietor: Adolor Corporation, Exton, PA 19341-1127 (US)
(72) Inventor: FARRAR, John, J., Chester Springs, PA 19425 (US); SCHIED, Peter, J., Southampton, PA 18966 (US); SCHMIDT, William, K., Wilmington DE 19810 (US); CARPENTER, Randall, L., Waban, MA 02168 (US)
(74) Representative: W.P. Thompson & Co.
(86) International application number: PCT/US2000/042313
(87) International publication number: WO 2001/042207

(56) References cited:
- WO-A1-83/03197
- WO-A1-99/22737
- US-A- 3 723 440
- US-A- 4 176 186
- US-A- 4 489 079
- US-A- 4 730 048
- US-A- 4 806 556
- US-A- 5 250 542
- ZIMMERMAN ET AL.: "Discovery of a potent, peripherally selective..." J.MED.CHEM., vol. 37, 1994, pages 2262-2265, XP002237983
- BOTROS ET AL.: "Opiod agonist and antagonist activities..." J.MED.CHEM., vol. 32, 1989, pages 2068-2071, XP002237984
- DATABASE BIOSIS [Online] BIOSCIENCES INFORMATION SERVICE, PHILADELPHIA, PA, US; 1982 KROMER W ET AL: "DIFFERENTIAL EFFECTS OF SKF-10047 N ALLYL NORMETAZOCINE ON PERISTALSIS AND LONGITUDINAL MUSCLE CONTRACTIONS OF THE ISOLATED GUINEA-PIG ILEUM" Database accession no. PREV198376067249 XP002238052 & NAUNYN-SCHMIEDEBERG'S ARCHIVES OF PHARMACOLOGY, vol. 321, no. 3, 1982, pages 218-222, ISSN: 0028-1298
- SCHAPIRA M ET AL: "THE CURRENT STATUS OF GASTRIC PROKINETIC DRUGS" ACTA GASTRO-ENTEROLOGICA BELGICA, ACTA MEDICA BELGICA, BRUSSELS, BE, vol. 53, no. 4, July 1990 (1990-07), pages 446-457, XP001041609 ISSN: 0001-5644
- MACK D.J.: 'Paralytic ileus: response to naloxone' BRITISH JOURNAL OF SURGERY vol. 76, no. 10, October 1989, XP002939468
- SCHANG ET AL.: 'Beneficial effects of naloxone in a patient with intestinal pseudoobstruction' AMERICAN JOURNAL OF GASTROENTEROLOGY vol. 80, no. 6, 1985, XP002939469
- WORLD PHARMACEUTICAL NEWS, 12 February 1999 (1999-02-12),
- PR NEWSWIRE, PAGE 0616NYTU019, 16 June 1998 (1998-06-16),
- ZIMMERMAN ET AL.: DRUGS OF THE FUTURE, vol. 19, 1994, pages 1078-1083,
- WERNER ET AL.: J.ORG.CHEM., vol. 61, - 1996 pages 587-597,
- "Harrisson's Principles of Internal Medicine, 13th ed., pages 1418-1422 and 1431" 1994, MCGRAW HILL INC
- RESNICK ET AL.: AM.J.GASTROENTEROLOGY, vol. 92, - 1997 pages 751-762,
- RESNICK ET AL.: AM.J.GASTROENTEROLOGY, vol. 92, 1997, pages 934-940,
- "Dorland's Illlustrated Medical Dictionary, 28th ed.,pages 819 and 1265" W.B. SAUNDERS COMPANY
- "Declaration of Dr. W. Schmidt" 9 October 2003 (2003-10-09),
- FOSS ET AL.: J.CLIN.PHARMACOL., vol. 37, pages 25-30,
- FOSS ET AL.: CLIN.PHARMACOL.THER., vol. 61, - 1997 pages 467-475,

## Description

The present invention relates to novel methods for the treatment and prevention of ileus. More particularly, the present invention relates to novel methods for the treatment and prevention of ileus by using peripheral mu opioid antagonist compounds.

### Background of the Invention

It is well known that opioid drugs target three types of endogenous opioid receptors (*i*.*e*., mu, delta and kappa receptors) in biological systems. Many opiates, such as morphine, are mu opioid agonists that are often used as analgesics for the treatment of severe pain due to their activation of mu opioid receptors in the brain and central nervous system (CNS). Opioid receptors are, however, not limited to the CNS, and may be found in other tissues throughout the body. A number of side effects of opioid drugs may be caused by activation of these peripheral receptors. For example, administration of mu opioid agonists often results in intestinal dysfunction due to the large number of receptors in the wall of the gut (Wittert, G., Hope, P. and Pyle, D., Biochemical and Biophysical Research Communications 1996, 218, 877-881; Bagnol, D., Mansour, A., Akil, A. and Watson, S.J., Neuroscience 1997, 81, 579-591). Specifically, opioids are generally known to cause nausea and vomiting as well as inhibition of normal propulsive gastrointestinal function in animals and man (Reisine, T., and Pasternak, G., Goodman & Gilman's The Pharmacological Basis of Therapeutics Ninth Edition 1996, 521-555) resulting in side effects such as, for example, constipation.

Recent evidence has indicated that naturally occurring endogenous opioid compounds may also affect propulsive activity in the gastrointestinal (GI) tract. Met-enkephalin, which activates mu and delta receptors in both the brain and gut, is one of several neuropeptides found in the GI tract (Koch, T.R., Carney, J.A., Go, V.L., and Szurszewski, J.H., Digestive Diseases and Sciences 1991, 36, 712-728). Additionally, receptor knockout techniques have shown that mice lacking mu opioid receptors may have faster GI transit times than wild-type mice, suggesting that endogenous opioid peptides may tonically inhibit GI transit in normal mice (Schuller, A.G.P., King, M., Sherwood, A.C., Pintar, J.E., and Pasternak, G.W., Society of Neuroscience Abstracts 1998, 24, 524). Studies have shown that opioid peptides and receptors located throughout the GI tract may be involved in normal regulation of intestinal motility and mucosal transport of fluids in both animals and man (Reisine, T., and Pasternak, G., Goodman & Gilman's The Pharmacological Basis of Therapeutics Ninth Edition 1996, 521-555). Other studies show that the sympathetic nervous system may be associated with endogenous opioids and control of intestinal motility (Bagnol, D., Herbrecht, F., Jule, Y., Jarry, T., and Cupo, A., Regul. Pept. 1993, 47, 259-273). The presence of endogenous opioid compounds associated with the GI tract suggests that an abnormal physiological level of these compounds may lead to bowel dysfunction.

It is a common problem for patients having undergone surgical procedures, especially surgery of the abdomen, to suffer from a particular bowel dysfunction called post-surgical (or post-operative) ileus. "Ileus", as used herein, refers to the obstruction of the bowel or gut, especially the colon. *See, e.g.,* Dorland's Illustrated Medical Dictionary, p. 816, 27th ed. (W.B. Saunders Company, Philadelphia 1988). Ileus should be distinguished from constipation, which refers to infrequent or difficulty in evacuating the feces. *See, e.g.,* Dorland's Illustrated Medical Dictionary, p. 375, 27th ed. (W.B. Saunders Company, Philadelphia 1988). Ileus may be diagnosed by the disruption of normal coordinated movements of the gut, resulting in failure of the propulsion of intestinal contents. *See, e.g*., Resnick, J. Am. J. of Gastroenterology 1997, 92, 751 and Resnick, J. Am. J. of Gastroenterology, 1997, 92, 934. In some instances, particularly following surgery, including surgery of the abdomen, the bowel dysfunction may become quite severe, lasting for more than a week and affecting more than one portion of the GI tract. This condition is often referred to as post-surgical (or post-operative) paralytic ileus and most frequently occurs after laparotomy (see Livingston, E.H. and Passaro, E.D. Jr. Digestive Diseases and Sciences 1990, 35, 121). Similarly, post-partum ileus is a common problem for women in the period following childbirth, and is thought to be caused by similar fluctuations in natural opioid levels as a result of birthing stress.

Gastrointestinal dysmotility associated with post-surgical ileus is generally most severe in the colon and typically lasts for 3 to 5 days. The administration of opioid analgesics to a patient after surgery may often contribute to bowel dysfunction, thereby delaying recovery of normal bowel function. Since virtually all patients receive opioid analgesics, such as morphine or other narcotics for pain relief after surgery, particularly major surgery, current post-surgical pain treatment may actually slow recovery of normal bowel function, resulting in a delay in hospital discharge and increasing the cost of medical care.

Post-surgical ileus may also occur in the absence of exogenous opioid agonists. It would be of benefit to inhibit the natural activity of endogenous opioids during and/or after periods of biological stress such as surgery and childbirth so that ileus and related forms of bowel dysfunction can be prevented or treated. Currently, therapies for ileus include functional stimulation of the intestinal tract, stool softeners, laxatives, lubricants, intravenous hydration, and nasogastric decompression. These prior art methods suffer from drawbacks, for example, as lacking specificity for post-surgical or post-partum ileus. And these prior art methods offer no means for prevention. If ileus could be prevented, hospital stays, recovery times, and medical costs would be significantly decreased in addition to the benefit of minimizing patient discomfort. Thus, drugs which selectively act on opioid receptors in the gut would be ideal candidates for preventing and/or treating post-surgical and post-partum ileus. Of those, drugs that do not interfere with the effects of opioid analgesics in the CNS would be of special benefit in that they may be administered simultaneously for pain management with limited side effects.

Peripheral opioid antagonists that do not cross the blood-brain barrier into the CNS are known in the literature and have been tested in relation to their activity on the GI tract. In U.S. Patent Nos. 5,250,542, 5,434,171, 5,159,081, and 5,270,328, peripherally selective piperidine-N-alkylcarboxylate opioid antagonists are described as being useful in the treatment of idiopathic constipation, irritable bowel syndrome and opioid-induced constipation. Also, U.S. Patent No. 4,176,186 describes quaternary derivatives of noroxymorphone (*i*.*e*., methylnaltrexone) that are said to prevent or relieve the intestinal immobility side-effect of narcotic analgesics without reducing analgesic effectiveness. U.S. Patent No. 5,972,954 describes the use of methylnaltrexone, enteric coated methylnaltrexone, or other quaternary derivatives of noroxymorphone for preventing and/or treating opioid- and/or nonopioid-induced side effects associated with opioid administration.

General opioid antagonists such as naloxone and naltrexone have also been implicated as being useful in the treatment of GI tract dysmotility. For example, U.S. Patent No. 4,987,126 and Kreek, M.J. Schaefer, R.A., Hahn, E.F., Fishman, J. Lancet 1983, 1(8319), 261 disclose naloxone and other morphinan-based opioid antagonists (*i*.*e*., naloxone, naltrexone) for the treatment of idiopathic gastrointestinal dysmotility. In addition, naloxone has been shown to effectively treat non-opioid induced bowel obstruction, implying that the drug may act directly on the GI tract or in the brain (Schang, J.C., Devroede, G. Am J. Gastroenerol. 1985, 80(6), 407). Furthermore, it has been implicated that naloxone may provide therapy for paralytic ileus (Mack, D.J. Fulton, J.D. Br. J. Surg. 1989, 76(10), 1101). However, it is well known that activity of naloxone and related drugs is not limited to peripheral systems and may interfere with the analgesic effects of opioid narcotics.

WO99/22737 discloses a method for preventing or treating opioid induced side effects including gastrointestinal dysfunction and non-opioid induced changes in gastrointestinal motility. The method comprises administering methylnaltrexone or another quaternary derivative of noroxymorphone to a patient prior to the administration of an opioid or after the onset of side effects induced by the administration of an opioid.

World Pharmaceutical News of 12 February 1999 discloses a proposed Phase II trial of a specific oral peripheral mu antagonist, and refers to a potential application in the treatment of post-surgical ileus.

Inasmuch as post-surgical and post-partum ileus, for example, are common illnesses that add to the cost of health care and as yet have no specific treatments, there is a need for a specific and effective remedy. The majority of currently known opioid antagonist therapies are not peripherally selective and have the potential for undesirable side effects resulting from penetration into the CNS. Given the estimated 21 million inpatient surgeries and 26 outpatient surgeries each year, and an estimate of 4.7 million patients experiencing post-surgical ileus, methods involving opioid antagonists that are not only specific for peripheral systems, but specific for the gut, are desirable for treating post-surgical and post-partum ileus. The present invention is directed to this, as well as other important ends.

### Summary of the Invention

Accordingly, the present invention is directed to a compound of Formula II or a pharmaceutically acceptable salt, hydrate or N-oxide thereof 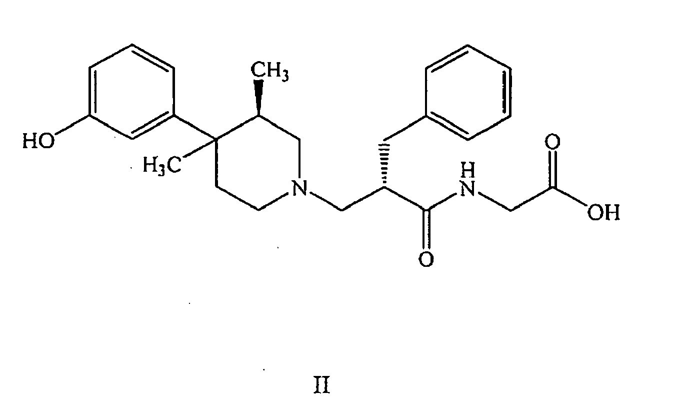 for use in treating or preventing ileus.

These and other aspects of the invention will become more apparent from the following detailed description.

### Brief Description of the Drawings

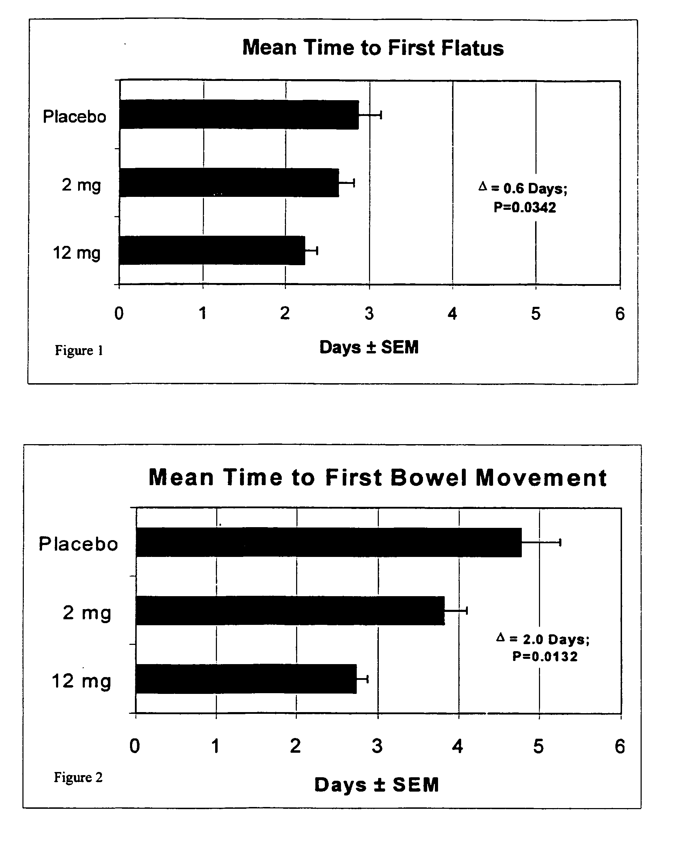

Figures 1 to 5 are graphical representations of studies on the treatment of ileus employing methods according to an embodiment of the present invention.

### Detailed Description of the Invention

As employed above and throughout the disclosure, the following terms, unless otherwise indicated, shall be understood to have the following meanings.

"Alkyl" refers to an aliphatic hydrocarbon group which may be straight, branched or cyclic having from 1 to about 10 carbon atoms in the chain, and all combinations and subcombinations of ranges therein. "Branched" refers to an alkyl group in which a lower alkyl group, such as methyl, ethyl or propyl, is attached to a linear alkyl chain. In certain preferred embodiments, the alkyl group is a C₁-C₅ alkyl group, *i*.*e*., a branched or linear alkyl group having from 1 to about 5 carbons. In other preferred embodiments, the alkyl group is a C₁-C₃ alkyl group, *i*.*e*., a branched or linear alkyl group having from 1 to about 3 carbons. Exemplary alkyl groups include methyl, ethyl, n-propyl, isopropyl, butyl, isobutyl, sec-butyl, tert-butyl, pentyl, hexyl, heptyl, octyl, nonyl and decyl. "Lower alkyl" refers to an alkyl group having 1 to about 6 carbon atoms. Preferred alkyl groups include the lower alkyl groups of 1 to about 3 carbons.

"Alkenyl" refers to an alkyl group containing at least one carbon-carbon double bond and having from 2 to about 10 carbon atoms in the chain, and all combinations and subcombinations of ranges therein. In certain preferred embodiments, the alkenyl group is a C₂-C₁₀ alkyl group, *i*.*e*., a branched or linear alkenyl group having from 2 to about 10 carbons. In other preferred embodiments, the alkenyl group is a C₂-C₆ alkenyl group, *i*.*e*., a branched or linear alkenyl group having from 2 to about 6 carbons. In still other preferred embodiments, the alkenyl group is a C₃-C₁₀ alkenyl group, *i*.*e*., a branched or linear alkenyl group having from about 3 to about 10 carbons. In yet other preferred embodiments, the alkenyl group is a C₂-C₅ alkenyl group, *i*.*e*., a branched or linear alkenyl group having from 2 to about 5 carbons. Exemplary alkenyl groups include, for example, vinyl, propenyl, butenyl, pentenyl hexenyl, heptenyl, octenyl, nonenyl and decenyl groups.

"Alkylene" refers to a straight or branched bivalent aliphatic hydrocarbon group having from 1 to about 6 carbon atoms, and all combinations and subcombinations of ranges therein. The alkylene group may be straight, branched or cyclic. Exemplary alkylene groups include, for example, methylene (-CH₂-), ethylene (-CH₂CH₂-) and propylene (-(CH₂)₃-). There may be optionally inserted along the alkylene group one or more oxygen, sulphur or optionally substituted nitrogen atoms, wherein the nitrogen substituent is alkyl as described previously. Preferred alkylene groups have from about 1 to about 4 carbons.

"Alkenylene" refers to an alkylene group containing at least one carbon-carbon double bond. Exemplary alkenylene groups include, for example, ethenylene (-CH=CH-) and propenylene (-CH=CHCH₂-). Preferred alkenylene groups have from 2 to about 4 carbons.

"Cycloalkyl" refers to any stable monocyclic or bicyclic ring having from about 3 to about 10 carbons, and all combinations and subcombinations of ranges therein. In preferred embodiments, the cycloalkyl group is a C₃-C₈ cycloalkyl group, *i*.*e*., a cycloalkyl group having from about 3 to about 8 carbons, with C₃-C₆ cycloalkyl groups, i.e., cycloalkyl groups having from about 3 to about 6 carbons being more preferred. The cycloalkyl group may be optionally substituted with one or more cycloalkyl group substituents. Preferred cycloalkyl group substituents include alkyl, preferably C₁-C₃ alkyl, alkoxy, preferably C₁-C₃ alkoxy, or halo. Exemplary cycloalkyl groups include, for example, cyclopropyl, cyclobutyl, cyclopentyl, cyclohexyl, cycloheptyl and cyclooctyl groups.

"Cycloalkyl-substituted alkyl" refers to a linear alkyl group, preferably a lower alkyl group, substituted at a terminal carbon with a cycloalkyl group, preferably a C₃-C₈ cycloalkyl group. Typical cycloalkyl-substituted alkyl groups include cyclohexylmethyl, cyclohexylethyl, cyclopentylethyl, cyclopentylpropyl, cyclopropylmethyl and the like.

"Cycloalkenyl" refers to an olefinically unsaturated cycloalkyl group having from about 4 to about 10 carbons, and all combinations and subcombinations of ranges therein. In preferred embodiments, the cycloalkenyl group is a C₅-C₈ cycloalkenyl group, *i.e.,* a cycloalkenyl group having from about 5 to about 8 carbons.

"Alkoxy" refers to an alkyl-O- group where alkyl is as previously described. Exemplary alkoxy groups include, for example, methoxy, ethoxy, propoxy, butoxy and heptoxy.

"Alkoxy-alkyl" refers to an alkyl-O-alkyl group where alkyl is as previously described.

"Acyl" means an alkyl-CO- group wherein alkyl is as previously described. Preferred acyl groups comprise lower alkyl groups, such as alkyl of about 1 to about 3 carbons. Exemplary acyl groups include acetyl, propanoyl, 2-methylpropanoyl, butanoyl and palmitoyl.

"Aryl" refers to an aromatic carbocyclic radical containing from about 6 to about 10 carbons, and all combinations and subcombinations of ranges therein. The phenyl group may be optionally substituted with one or two or more aryl group substituents. Preferred aryl group substituents include alkyl groups, preferably C₁-C₂ alkyl groups. Exemplary aryl groups include phenyl and naphthyl.

"Aryl-substituted alkyl" refers to an linear alkyl group, preferably a lower alkyl group, substituted at a terminal carbon with an optionally substituted aryl group, preferably an optionally substituted phenyl ring. Exemplary aryl-substituted alkyl groups include, for example, phenylmethyl, phenylethyl and 3-(4-methylphenyl)propyl.

"Heterocyclic" refers to a monocyclic or multicylic ring system carbocyclic radical containing from about 4 to about 10 members, and all combinations and subcombinations of ranges therein, wherein one or more of the members is an element other than carbon, for example, nitrogen, oxygen or sulfur. The heterocyclic group may be aromatic or nonaromatic. Exemplary heterocyclic groups include, for example, pyrrole and piperidine groups.

"Halo" refers to fluoro, chloro or bromo.

"Effective amount" refers to an amount of a compound as described herein that may be therapeutically effective to prevent or treat the symptoms of particular disorder. Such disorders include, but are not limited to, those pathological disorders associated with ileus, wherein the treatment or prevention comprises, for example, inhibiting the activity thereof by contacting cells, tissues or receptors with compounds of the present invention.

"Pharmaceutically acceptable" refers to those compounds, materials, compositions, and/or dosage forms which are, within the scope of sound medical judgment, suitable for contact with the tissues of human beings and animals without excessive toxicity, irritation, allergic response, or other problem complications commensurate with a reasonable benefit/risk ratio.

"Pharmaceutically acceptable salts" refer to derivatives of the disclosed compounds wherein the parent compound is modified by making acid or base salts thereof. Examples of pharmaceutically acceptable salts include, but are not limited to, mineral or organic acid salts of basic residues such as amines; alkali or organic salts of acidic residues such as carboxylic acids; and the like. The pharmaceutically acceptable salts include the conventional non-toxic salts or the quaternary ammonium salts of the parent compound formed, for example, from non-toxic inorganic or organic acids. For example, such conventional non-toxic salts include those derived from inorganic acids such as hydrochloric, hydrobromic, sulfuric, sulfamic, phosphoric, nitric and the like; and the salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxybenzoic, fumaric, toluenesulfonic, methanesulfonic, ethane disulfonic, oxalic, isethionic, and the like.

Certain acidic or basic compounds of the present invention may exist as zwitterions. All forms of the compounds, including free acid, free base and zwitterions, are contemplated to be within the scope of the present invention.

"Patient" refers to animals, including mammals, preferably humans.

The present invention is directed to compounds for the treatment or prevention of ileus. Different types of ileus may be treated and/or prevented using the compounds of the present invention. The present compounds are particularly suitable for treating and/or preventing post-surgical ileus and post-partum ileus. "Post-surgical ileus", which may follow surgery such as laparotomy, may be characterized by such symptoms as, for example, obstruction of the gut, particularly in the colon, resulting in nausea, vomiting, lack of passage of flatus and/or stools, abdominal distention and lack of bowel sounds. This condition generally lasts from about 3 to about 5 days, but may endure longer, including up to about one week. Longer durations are generally characteristic of a more severe form of ileus, termed post-surgical paralytic ileus, which may affect other portions of the GI tract in addition to the colon. "Post-partum ileus" generally refers to obstruction of the gut, particularly the colon, following parturition. Both natural and surgically-assisted procedures during parturition may lead to post-partum ileus treated by the present invention. Symptoms of post-partum ileus and post-surgical ileus are similar.

The compounds of the present invention may be used to treat patients who are also being administered compounds that may slow gut motility including, for example, opiates and/or opioids, such as opioid analgesics, prior to, during, and subsequent to the onset of ileus. The administration of such opiate or opioid compounds may induce bowel dysfunction which, in turn, may delay recovery from ileus, including postoperative ileus. The compounds of the present invention may also be used to treat patients who have not received any exogenous opiates and/or opioids.

While not intending to be bound by any theory or theories of operation, it is contemplated that ileus, particularly post-surgical ileus and post-partum ileus, may result from stress-induced abnormal levels of endogenous opioid compounds. Administration of a mu opioid antagonist according to the methods of the present invention may block interaction of the endogenous opioid compounds with the mu receptors in the gut, thereby preventing and/or inhibiting ileus.

The compound of formula (II) has low solubility in water except at low or high pH conditions. Zwitterionic character may be inherent to the compound, and may impart desirable properties such as poor systemic absorption and sustained local affect on the gut following oral administration.

The compounds employed in the present invention may be prepared in a number of ways well known to those skilled in the art. The compounds can be synthesized, for example, by the methods described below, or variations thereon as appreciated by the skilled artisan. All processes disclosed in association with the present invention are contemplated to be practiced on any scale, including milligram, gram, multigram, kilogram, multikilogram or commercial industrial scale.

As discussed in detail above, compounds employed in the present methods contain one or more asymmetrically substituted carbon atoms, and may be isolated in optically active or racemic forms. Thus, all chiral, diastereomeric, racemic forms and all geometric isomeric forms of a structure are intended, unless the specific stereochemistry or isomeric form is specifically indicated. It is well known in the art how to prepare and isolate such optically active forms. For example, mixtures of stereoisomers may be separated by standard techniques including, but not limited to, resolution of racemic forms, normal, reverse-phase, and chiral chromatography, preferential salt formation, recrystallization, and the like, or by chiral synthesis either from chiral starting materials or by deliberate synthesis of target chiral centers.

As will be readily understood, functional groups present may contain protecting groups during the course of synthesis. Protecting groups are known *per se* as chemical functional groups that can be selectively appended to and removed from functionalities, such as hydroxyl groups and carboxyl groups. These groups are present in a chemical compound to render such functionality inert to chemical reaction conditions to which the compound is exposed. Any of a variety of protecting groups may be employed with the present invention. Preferred protecting groups include the benzyloxycarbonyl group and the tert-butyloxycarbonyl group. Other preferred protecting groups that may be employed in accordance with the present invention may be described in Greene, T.W. and Wuts, P.G.M., Protective Groups in Organic Synthesis 2d. Ed., Wiley & Sons, 1991.

Piperidine-N-alkylcarboxylate compounds according to the present invention may be synthesized employing methods taught, for example, in U.S. Patent Nos. 5,250,542, 5,434,171, 5,159,081, and 5,270,328. For example, the 3-substituted-4-methyl-4-(3-hydroxy- or alkanoyloxyphenyl)piperidine derivatives employed as starting materials in the synthesis of the present compounds may be prepared by the general procedure taught in U.S. Patent No. 4,115,400 and U.S. Patent No. 4,891,379. The starting material for the synthesis of compounds described herein, (3R,4R)-4-(3-hydroxypheny)-3,4-dimethylpiperidine, may be prepared by the procedures described in U.S. Patent No. 4,581,456, but adjusted as described such that the β-stereochemistry is preferred.

The first step of the process may involves the formation of the 3-alkoxyphenyllithium reagent by reacting 3-alkoxybromobenzene with an alkyllithium reagent. This reaction may be performed under inert conditions and in the presence of a suitable non-reactive solvent such as dry diethyl ether or preferably dry tetrahydrofuran. Preferred alkyllithium reagents used in this process are n-butyllithium, and especially sec-butyllithium. Generally, approximately an equimolar to slight excess of alkyllithium reagent may be added to the reaction mixture. The reaction may be conducted at a temperature of from about -20°C and about -100°C, more preferably from about -50°C to about -55°C.

Once the 3-alkoxyphenyllithium reagent has formed, approximately an equimolar quantity of a 1-alkyl-4-piperidone may be added to the mixture while maintaining the temperature between -20°C and -100°C. The reaction is typically complete after about 1 to 24 hours. At this point, the reaction mixture may be allowed to gradually warm to room temperature. The product may be isolated by the addition to the reaction mixture of a saturated sodium chloride solution to quench any residual lithium reagent. The organic layer may be separated and further purified if desired to provide the appropriate 1-alkyl-4-(3-alkoxyphenyl)piperidinol derivative.

The dehydration of the 4-phenylpiperidinol prepared above may be accomplished with a strong acid according to well known procedures. While dehydration occurs in various amounts with any one of several strong acids such as hydrochloric acid, hydrobromic acid, and the like, dehydration is preferably conducted with phosphoric acid, or especially p-toluenesulfonic acid in toluene or benzene. This reaction may be typically conducted under reflux conditions, more generally from about 50°C and 150°C. The product thus formed may be isolated by basifying an acidic aqueous solution of the salt form of the product and extracting the aqueous solution with a suitable water immiscible solvent. The resulting residue following evaporation can then be further purified if desired.

The 1-alkyl-4-methyl-4-(3-alkoxyphenyl)tetrahydropyridine derivatives may be prepared by a metalloenamine alkylation. This reaction is preferably conducted with n-butyllithium in tetrahydrofuran (THF) under an inert atmosphere, such as nitrogen or argon. Generally, a slight excess of n-butyllithium may be added to a stirring solution of the 1-alkyl-4-(3-alkoxyphenyl)-tetrahydropyridine in THF cooled to a temperature in the range of from about -50°C to about 0°C, more preferably from about -20°C to -10°C. This mixture may be stirred for approximately 10 to 30 minutes followed by the addition of approximately from 1.0 to 1.5 equivalents of methyl halide to the solution while maintaining the temperature of the reaction mixture below 0°C. After about 5 to 60 minutes, water may be added to the reaction mixture and the organic phase may be collected. The product can be purified according to standard procedures, but the crude product is preferably purified by either distilling it under vacuum or slurrying it in a mixture of hexane:ethyl acetate (65:35, v:v) and silica gel for about two hours. According to the latter procedure, the product may be then isolated by filtration followed by evaporating the filtrate under reduced pressure.

The next step in the process may involve the application of the Mannich reaction of aminomethylation to non-conjugated, endocyclic enamines. This reaction is preferably carried out by combining from about 1.2 to 2.0 equivalents of aqueous formaldehyde and about 1.3 to 2.0 equivalents of a suitable secondary amine in a suitable solvent. While water may be the preferred solvent, other non-nucleophilic solvents, such as acetone and acetonitrile can also be employed in this reaction. The pH of this solution may be adjusted to approximately 3.0 to 4.0 with an acid that provides a non-nucleophilic anion. Examples of such acids include sulfuric acid, the sulfonic acids such as methanesulfonic acid and p-toluenesulfonic acid, phosphoric acid, and tetrafluoroboric acid, with sulfuric acid being preferred. To this solution may be added one equivalent of a 1-alkyl-4-methyl-4-(3-alkoxyphenyl)tetrahydropyridine, typically dissolved in aqueous sulfuric acid, and the pH of the solution may be readjusted with the non-nucleophilic acid or a suitable secondary amine. The pH is preferably maintained in the range of from about 1.0 to 5.0, with a pH of about 3.0 to 3.5 being more preferred during the reaction. The reaction is substantially complete after about 1 to 4 hours, more typically about 2 hours, when conducted at a temperature in the range of from about 50°C to about 80°C, more preferably about 70°C. The reaction may then be cooled to approximately 30°C, and added to a sodium hydroxide solution. This solution may then be extracted with a water immiscible organic solvent, such as hexane or ethyl acetate, and the organic phase, following thorough washing with water to remove any residual formaldehyde, may be evaporated to dryness under reduced pressure.

The next step of the process may involve the catalytic hydrogenation of the prepared 1-alkyl-4-methyl-4-(3-alkoxyphenyl)-3-tetrahydropyridinemethanamine to the corresponding trans-1-alkyl-3,4-dimethyl-4-(3-alkoxyphenyl)piperidine. This reaction actually occurs in two steps. The first step is the hydrogenolysis reaction wherein the exo C-N bond is reductively cleaved to generate the 3-methyltetrahydropyridine. In the second step, the 2,3-double bond in the tetrahydropyridine ring is reduced to afford the desired piperidine ring.

Reduction of the enamine double bond introduced the crucial relative stereochemistry at the 3 and 4 carbon atoms of the piperidine ring. The reduction generally does not occur with complete stereoselectivity. The catalysts employed in the process may be chosen from among the various palladium and preferably platinum catalysts.

The catalytic hydrogenation step of the process is preferably conducted in an acidic reaction medium. Suitable solvents for use in the process include the alcohols, such as methanol or ethanol, as well as ethyl acetate, tetrahydrofuran, toluene, hexane, and the like.

Proper stereochemical outcome may be dependent on the quantity of catalyst employed. The quantity of catalyst required to produce the desired stereochemical result may be dependent upon the purity of the starting materials in regard to the presence or absence of various catalyst poisons.

The hydrogen pressure in the reaction vessel may not be critical but can be in the range of from about 34.5 kPa to 1.38 mPa (5 to 200 psi). Concentration of the starting material by volume is preferably around 20 mL of liquid per gram of starting material, although an increased or decreased concentration of the starting material can also be employed. Under the conditions specified herein, the length of time for the catalytic hydrogenation may not be critical because of the inability for over-reduction of the molecule. While the reaction can continue for up to 24 hours or longer, it may not be necessary to continue the reduction conditions after the uptake of the theoretical two moles of hydrogen. The product may then be isolated by filtering the reaction mixture for example through infusorial earth, and evaporating the filtrate to dryness under reduced pressure. Further purification of the product thus isolated may not be necessary and preferably the diastereomeric mixture may be carried directly on to the following reaction.

The alkyl substituent may be removed from the 1-position of the piperidine ring by standard dealkylation procedures. Preferably, a chloroformate derivative, especially the vinyl or phenyl derivatives, may be employed and removed with acid. Next, the prepared alkoxy compound may be dealkylated to the corresponding phenol. This reaction may be generally carried out by reacting the compound in a 48% aqueous hydrobromic acid solution. This reaction may be substantially complete after about 30 minutes to 24 hours when conducted at a temperature of from about 50°C to about 150°C, more preferably at the reflux temperature of the reaction mixture. The mixture may then be worked up by cooling the solution, followed by neutralization with base to an approximate pH of 8. This aqueous solution may be extracted with a water immiscible organic solvent. The residue following evaporation of the organic phase may then be used directly in the following step.

The compounds employed as starting materials to the compounds of the invention can also be prepared by brominating the 1-alkyl-4-methyl-4-(3-alkoxyphenyl)-3-tetrahydropyridinemethanamine at the 3-position, lithiating the bromo compound thus prepared, and reacting the lithiated intermediate with a methylhalide, such as methyl bromide to provide the corresponding 1-alkyl-3,4-dimethyl-4-(3-alkoxyphenyl)tetrahydropyridinemethanamine. This compound may then be reduced and converted to the starting material as indicated above.

As noted above, the compounds of the present invention can exist as the individual stereoisomers. Preferably reaction conditions are adjusted as disclosed in U.S. Patent No. 4,581,456 or as set forth in Example 1 of U.S. Patent No. 5,250,542 to be substantially stereoselective and provide a racemic mixture of essentially two enantiomers. These enantiomers may then be resolved. A procedure which may be employed to prepare the resolved starting materials used in the synthesis of these compounds includes treating a racemic mixture of alkyl-3,4-dimethyl-4-(3-alkoxyphenyl)piperidine with either (+)- or (-)-ditoluoyl tartaric acid to provide the resolved intermediate. This compound may then be dealkylated at the 1-position with vinyl chloroformate and finally converted to the desired 4-(3-hydroxyphenyl)piperidine isomer.

As will be understood by those skilled in the art, the individual enantiomers of the invention can also be isolated with either (+) or (-) dibenzoyl tartaric acid, as desired, from the corresponding racemic mixture of the compounds of the invention. Preferably the (+)-trains enantiomer is obtained.

Intermediates can be prepared by reacting a 3,4-alkyl-substituted-4-(3-hydroxyphenyl)piperidine with a compound of the formula LCH₂(CH₂)ₙ₋₁CHR³C(O)E where L is a leaving group such as chlorine, bromine or iodine, E is a carboxylic acid, ester or amide, and R³ and n are as defined hereinabove. Preferably L may be chlorine and the reaction is carried out in the presence of a base to alkylate the piperidine nitrogen. For example 4-chloro-2-cyclohexylbutanoic acid, ethyl ester can be contacted with (3R,4R)-4-(3-hydroxyphenyl)-3,4-dimethylpiperidine to provide 4-[(3R,4R)-4-(3-hydroxyphenyl)-3,4-dimethyl-1-piperidine]butanoic acid, ethyl ester. Although the ester of the carboxylic acid may be preferred, the free acid itself or an amide of the carboxylic acid may be used.

In alternative synthesis, the substituted piperidine can be contacted with a methylene alkyl ester to alkylate the piperidine nitrogen. For example, 2-methylene-3-phenylproponic acid, ethyl ester can be contacted with a desired piperidine to provide 2-benzyl-3-piperidinepropanoic acid ethyl ester.

Another synthetic route can involve the reaction of a substituted piperidine with a haloalkylnitrile. The nitrile group of the resulting piperidine alkylnitrile can be hydrolyzed to the corresponding carboxylic acid.

With each of the synthetic routes, the resulting ester or carboxylic acid can be reacted with an amine or alcohol to provide modified chemical structures. In the preparation of amides, the piperidine-carboxylic acid or -carboxylic acid ester may be reacted with an amine in the presence of a coupling agent such as dicyclohexylcarbodiimide, boric acid, borane-trimethylamine, and the like. Esters can be prepared by contacting the piperidine-carboxylic acid with the appropriate alcohol in the presence of a coupling agent such as p-toluenesulfonic acid, boron trifluoride etherate or N,N'-carbonyldiimidazole. Alternatively, the piperidine-carboxylic acid chloride can be prepared using a reagent such as thionyl chloride, phosphorus trichloride, phosphorus pentachloride and the like. This acyl chloride can be reacted with the appropriate amine or alcohol to provide the corresponding amide or ester.

The compounds employed in the present invention may be administered by any means that results in the contact of the active agent with the agent's site of action in the body of a patient. The compounds may be administered by any conventional means available for use in conjunction with pharmaceuticals, either as individual therapeutic agents or in a combination of therapeutic agents. For example, they may be administered as the sole active agent in a pharmaceutical composition, or they can be used in combination with other therapeutically active ingredients including, for example, opioid analgesic agents.

The compounds are preferably combined with a pharmaceutical carrier selected on the basis of the chosen route of administration and standard pharmaceutical practice as described, for example, in Remington's Pharmaceutical Sciences (Mack Pub. Co., Easton, PA, 1980).

Compounds of the present invention can be administered to a mammalian host in a variety of forms adapted to the chosen route of administration, *e*.*g*., orally or parenterally. Parenteral administration in this respect includes administration by the following routes: intravenous, intramuscular, subcutaneous, rectal, intraocular, intrasynovial, transepithelial including transdermal, ophthalmic, sublingual and buccal; topically including ophthalmic, dermal, ocular, rectal, and nasal inhalation via insufflation aerosol.

The active compound may be orally administered, for example, with an inert diluent or with an assimilable edible carrier, or it may be enclosed in hard or soft shell gelatin capsules, or it may be compressed into tablets, or it may be incorporated directly with the food of the diet. For oral therapeutic administration, the active compound may be incorporated with excipient and used in the form of ingestible tablets, buccal tablets, troches, capsules, elixirs, suspensions, syrups, wafers, and the like. Such compositions and preparations should preferably contain at least 0.1 % of active compound. The percentage of the compositions and preparations may, of course, be varied and may conveniently be, for example, from about 2 to about 6% of the weight of the unit. The amount of active compound in such therapeutically useful compositions is preferably such that a suitable dosage will be obtained. Preferred compositions or preparations according to the present invention may be prepared so that an oral dosage unit form contains from about 0.1 to about 1000 mg of active compound.

The tablets, troches, pills, capsules and the like may also contain one or more of the following: a binder, such as gum tragacanth, acacia, corn starch or gelatin; an excipient, such as dicalcium phosphate; a disintegrating agent, such as corn starch, potato starch, alginic acid and the like; a lubricant, such as magnesium stearate; a sweetening agent such as sucrose, lactose or saccharin; or a flavoring agent, such as peppermint, oil of wintergreen or cherry flavoring. When the dosage unit form is a capsule, it may contain, in addition to materials of the above type, a liquid carrier. Various other materials may be present as coatings or to otherwise modify the physical form of the dosage unit. For instance, tablets, pills, or capsules may be coated with shellac, sugar or both. A syrup or elixir may contain the active compound, sucrose as a sweetening agent, methyl and propylparabens as preservatives, a dye and flavoring, such as cherry or orange flavor. Of course, any material used in preparing any dosage unit form is preferably pharmaceutically pure and substantially non-toxic in the amounts employed. In addition, the active compound may be incorporated into sustained-release preparations and formulations.

The active compound may also be administered parenterally or intraperitoneally. Solutions of the active compound as a free base or a pharmacologically acceptable salt can be prepared in water suitably mixed with a surfactant, such as hydroxypropylcellulose. A dispersion can also be prepared in glycerol, liquid polyethylene glycols and mixtures thereof and in oils. Under ordinary conditions of storage and use, these preparations may contain a preservative to prevent the growth of microorganisms.

The pharmaceutical forms suitable for injectable use include, for example, sterile aqueous solutions or dispersions and sterile powders for the extemporaneous preparation of sterile injectable solutions or dispersions. In all cases, the form is preferably sterile and fluid to provide easy syringability. It is preferably stable under the conditions of manufacture and storage and is preferably preserved against the contaminating action of microorganisms such as bacteria and fungi. The carrier may be a solvent or dispersion medium containing, for example, water, ethanol, polyol (for example, glycerol, propylene glycol, liquid polyethylene glycol and the like), suitable mixtures thereof, and vegetable oils. The proper fluidity can be maintained, for example, by the use of a coating, such as lecithin, by the maintenance of the required particle size in the case of a dispersion, and by the use of surfactants. The prevention of the action of microorganisms may be achieved by various antibacterial and antifungal agents, for example, parabens, chlorobutanol, phenol, sorbic acid, thimerosal and the like. In many cases, it will be preferable to include isotonic agents, for example, sugars or sodium chloride. Prolonged absorption of the injectable compositions may be achieved by the use of agents delaying absorption, for example, aluminum monostearate and gelatin.

Sterile injectable solutions may be prepared by incorporating the active compound in the required amount, in the appropriate solvent, with various of the other ingredients enumerated above, as required, followed by filtered sterilization. Generally, dispersions may be prepared by incorporating the sterilized active ingredient into a sterile vehicle that contains the basic dispersion medium and the required other ingredients from those enumerated above. In the case of sterile powders for the preparation of sterile injectable solutions, the preferred methods of preparation may include vacuum drying and the freeze drying technique which yield a powder of the active ingredient, plus any additional desired ingredient from the previously sterile-filtered solution thereof.

The therapeutic compounds of this invention may be administered to a patient alone or in combination with a pharmaceutically acceptable carrier. As noted above, the relative proportions of active ingredient and carrier may be determined, for example, by the solubility and chemical nature of the compound, chosen route of administration and standard pharmaceutical practice.

The dosage of the compounds of the present invention that will be most suitable for prophylaxis or treatment will vary with the form of administration, the particular compound chosen and the physiological characteristics of the particular patient under treatment. Generally, small dosages may be used initially and, if necessary, increased by small increments until the desired effect under the circumstances is reached. The therapeutic human dosage, based on physiological studies using rats, may generally range from about 0.01 mg to about 100 mg/kg of body weight per day, and all combinations and subcombinations of ranges therein. Alternatively, the therapeutic human dosage may be from about 0.4 mg to about 10 g or higher, and may be administered in several different dosage units from once to several times a day. Generally speaking, oral administration may require higher dosages.

Compounds of formula (II) have been characterized in opioid receptor binding assays and show preferential binding to mu opioid receptors. Studies in isolated tissues (guinea pig ileum and mouse vas deferens) have shown that these compounds may act as antagonists with no measurable agonist activity. Studies in animals have demonstrated that the present compounds may reverse constipation in morphine-dependent mice when administered orally or parenterally at very low doses, and do not block the analgesic actions of morphine unless given in hundred-fold or higher doses. Collectively, the data indicate that the compounds described herein may have a very high degree of peripheral selectivity. *See, e*.*g*., Zimmerman, D.M., et al., Drugs of the Future, 1994, 19(12), 1078-1083.

The invention is further described in the following example. The example, which is an actual example, is for illustrative purposes only, and is not to be construed as limiting the appended claims.

### Example

This example is directed to in vivo experiments in humans which demonstrate the effectiveness of the methods of the present invention.

A 78 patient Phase II clinical study was conducted which compared two doses (2 mg and 12 mg) of the compound of formula (II) versus placebo in patients undergoing partial colectomy or simple or radical hysterectomy surgical procedures. The results of this study are set forth in the following Tables 1 5o 4 and are depicted graphically in Figures 1 to 5.

**TABLE 1: MEAN TIME TO FIRST FLATUS**

| Dosage | Days | SEM (days) | Hours |
|---|---|---|---|
| 12 mg | 2.23 | 0.151 | 53.438 |
| 2 mg | 2.62 | 0.192 | 62.967 |
| Placebo | 2.86 | 0.275 | 68.675 |

| | | | |
|---|---|---|---|
| Δ = 0.63 | | | |

**TABLE 2: MEAN TIME TO FIRST BOWEL MOVEMENT**

| Dosage | Days | SEM (days) | Hours |
|---|---|---|---|
| 12 mg | 2.72 | 0.144 | 65.374 |
| 2 mg | 3.81 | 0.288 | 91.407 |
| Placebo | 4.77 | 0.488 | 114.406 |

| | | | |
|---|---|---|---|
| Δ = 2.04 | | | |

**TABLE 3: MEAN TIME TO SOLID DIET**

| Dosage | Days | SEM (days) | Hours |
|---|---|---|---|
| 12 mg | 2.60 | 0.139 | 62.515 |
| 2 mg | 3.35 | 0.247 | 80.298 |
| Placebo | 3.89 | 0.315 | 93.269 |

| | | | |
|---|---|---|---|
| Δ = 1.28 | | | |

**TABLE 4: MEAN TIME TO READY TO DISCHARGE**

| Dosage | Days | SEM (days) | Hours |
|---|---|---|---|
| 12 mg | 3.11 | 0.137 | 74.657 |
| 2 mg | 3.61 | 0.266 | 86.565 |
| Placebo | 3.94 | 0.277 | 94.536 |

| | | | |
|---|---|---|---|
| Δ = 0.83 | | | |

**TABLE 5: MEAN TIME TO ACTUAL DISCHARGE**

| Dosage | Days | SEM (days) | HOURS |
|---|---|---|---|
| 12 mg | 3.10 | 0.163 | 74.404 |
| 2 mg | 4.05 | 0.267 | 97.281 |
| Placebo | 4.54 | 0.344 | 109.071 |

| | | | |
|---|---|---|---|
| Δ = 1.44 | | | |

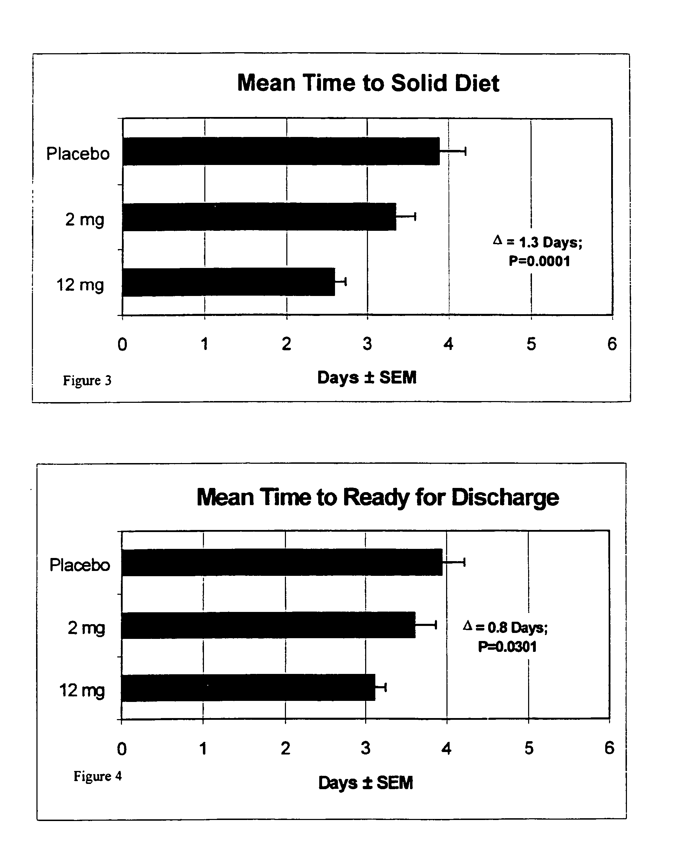

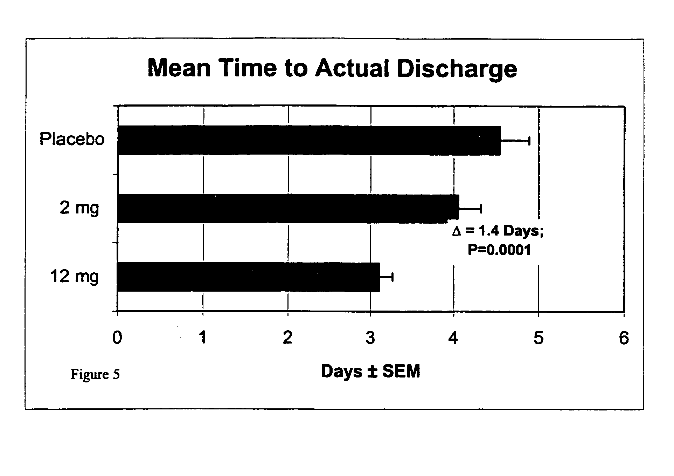

An analysis of the data in Tables 1 to 5 and Figures 1 to 5 shows a dose-dependent effect and that patients receiving a higher dose of the compound of formula (II) experienced shorter times to the following: (i) time to first flatus (P<0.04) (see Table 1 and Figure 1); (ii) time to first bowel movement (P<0.02) (see Table 2 and Figure 2); (iii) time to solid diet (P=0.0001) (see Table 3 and Figure 3); (iv) time to being ready for discharge from the hospital (P<0.04) (see Table 4 and Figure 4); and (v) time to discharge from the hospital (P=0.0001) (see Table 5 and Figure 5). The time to first flatus was reduced by 15 hours; all other measures were reduced by 24 hours or more. No patients experienced serious adverse side effects in this trial that were judged by the clinical investigator to be related to the activity of the compound of formula (II). The patients treated with the higher dose of the compound of formula (II) actually experienced fewer overall adverse effects than the patients in the placebo-treated group since compound (II) blocked the adverse gastrointestinal effects of morphine or other narcotic analgesics that were used for post- surgical pain relief. In particular, none of the patients receiving the higher dose of the compound of formula (II) experienced post-surgical vomiting compared to 23% in the placebo control group (P<0.03). Twenty seven percent of the patients receiving the higher dose of compound (II) experienced clinically relevant post- surgical nausea compared to 63% of the placebo control group (P=0.003). The compound of formula (II) also did not reduce the beneficial analgesic effects of systemic narcotics used in this trial. These results demonstrate that the compound of formula (II) may speed recovery of normal bowel function after surgery.

## Claims

1. A compound of Formula II or a pharmaceutically acceptable salt, hydrate or N-oxide thereof 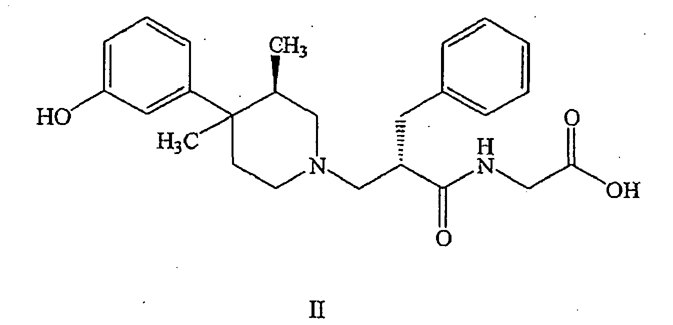 for use in treating or preventing ileus.

2. A compound as claimed in claim 1, wherein the ileus is selected from postsurgical ileus and postpartum ileus.

3. A compound as claimed in claim 2, wherein the ileus is postsurgical ileus.

4. A compound as claimed in claim 3, wherein said postsurgical ileus is postsurgical paralytic ileus.

5. A compound as claimed in any preceding claim, wherein the ileus is ileus of the colon.

6. A compound as claimed in any preceding claim, wherein the ileus occurs in the absence of exogenous opioid agonists.

7. A compound as claimed in claim 1, for use in treating or preventing ileus when administered with an opiate or an opioid.

8. A compound as claimed in claim 7, wherein said opiate or opioid comprises an opioid analgesic.

9. A compound as claimed in claim 8, wherein said opioid analgesic comprises a mu opioid agonist.

10. A compound as claimed in claim 1, for use in treating or preventing ileus when administered with a compound which slows gut motility.

## Patentansprüche

1. Verbindung der Formel II oder pharmazeutisch zulässiges Salz, Hydrat oder N-Oxid hiervon 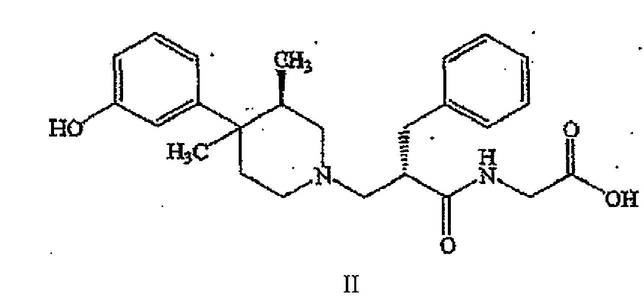 für die Verwendung zur Behandlung oder Prävention von Ileus.

2. Verbindung nach Anspruch 1, worin der Ileus ausgewählt ist von postoperativem Ileus und postpartalem Ileus.

3. Verbindung nach Anspruch 2, worin der Ileus postoperativer Ileus ist.

4. Verbindung nach Anspruch 3, worin der postoperative Ileus postoperativer paralytischer Ileus ist.

5. Verbindung nach einem der vorhergehenden Ansprüche, worin der Ileus ein Ileus des Dickdarms ist.

6. Verbindung nach einem der vorhergehenden Ansprüche, worin der Ileus in Abwesenheit von exogenen Opioidagonisten auftritt.

7. Verbindung nach Anspruch 1 für die Verwendung zur Behandlung oder Prävention von Ileus bei Verabreichung mit einem Opiat oder Opioid.

8. Verbindung nach Anspruch 7, worin das Opiat oder Opioid ein opioides Analgetikum umfasst.

9. Verbindung nach Anspruch 8, worin das opioide Analgetikum einen Mu-OpioidAgonisten umfasst.

10. Verbindung nach Anspruch 1 für die Verwendung zur Behandlung oder Prävention von Ileus bei Verabreichung mit einer Verbindung, die die Darmmotilität verlangsamt.

## Revendications

1. Composé de formule II ou sel, hydrate ou N-oxyde acceptable au plan pharmaceutique de ce dernier : 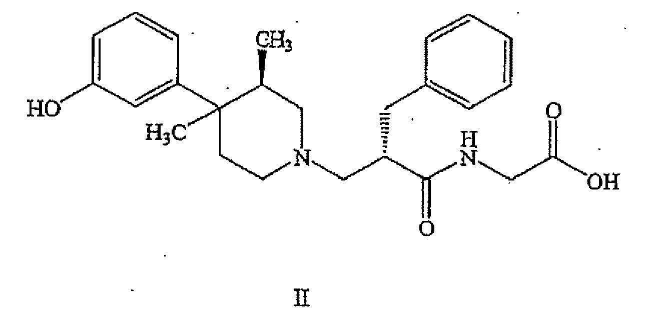 pour une utilisation dans le traitement ou dans la prévention d'un iléus.

2. Composé selon la revendication 1, dans lequel l'iléus est choisi parmi l'iléus post-chirurgical et l'iléus post-partum.

3. Composé selon la revendication 2, dans lequel l'iléus est un iléus post-chirurgical.

4. Composé selon la revendication 3, dans lequel l'iléus post-chirurgical est un iléus paralytique post-chirurgical.

5. Composé selon l'une quelconque des revendications précédentes, dans lequel l'iléus est un iléus du colon.

6. Composé selon l'une quelconque des revendications précédentes, dans lequel l'iléus survient en l'absence d'agonistes opioïdes exogènes.

7. Composé selon la revendication 1, pour une utilisation dans le traitement ou dans la prévention d'un iléus lorsqu'il est administré avec un opiacé ou un opioïde.

8. Composé selon la revendication 7, dans lequel ledit opiacé ou ledit opioïde comprend un analgésique opioïde.

9. Composé selon la revendication 8, dans lequel ledit analgésique opioïde comprend un agoniste opioïde de type mu.

10. Composé selon la revendication 1, pour une utilisation dans le traitement ou dans la prévention d'un iléus lorsqu'il est administré avec un composé qui ralentit la motilité de l'intestin.
